# EUROPEAN PATENT APPLICATION

(11) **EP 4 474 376 A1**
(43) Date of publication of application: **11.12.2024**
(21) Application number: 24177238.3
(22) Date of filing: 22.05.2024
(51) Int. Cl.: C07C 67/03, C07C 67/31, C07C 69/708, C07C 69/72, C11C 3/00

(54) **A METHOD FOR OBTAINING MICHAEL ADDITION DONORS FROM RAPESEED OIL**

(30) Priority: 23.05.2023 LV 230048
(71) Applicant: Sia Nedex, Liepaja 3401 (LV)
(72) Inventor: Kirpluks, Mikelis, 1021 Riga (LV); Abolins, Arnis, 2130 Dreilini (LV); Fridrihsone, Anda, 1039 Riga (LV); Pomilovskis, Ralfs, 3601 Ventspils (LV); Rundans, Maris, 3101 Tukums (LV)

(57) **Abstract**

The invention relates to a method for extracting Michael addition donors from rapeseed oil. For this purpose, rapeseed oil is converted into polyols by epoxidation followed by opening of the oxirane ring. The opening of oxirane rings is done with alcohol, methanol, 1.4-butanediol, diethylene glycol, trimethylolpropane. The resulting rapeseed polyols are converted to Michael addition donors via their acetoacetylation with *tert-butyl acetoacetate.* Michael addition donors synthesised from rapeseed oil have lower viscosity and higher functionality than prototype analogues synthesised from commercially available polyols.

## Description

### Field of invention

The invention relates to the use of rapeseed oil to obtain Michael donors which can be used to produce biopolymeric materials by means of the Michael nucleophilic addition reaction.

### Known state of the art

Fatty acids, in their diglyceride and triglyceride forms, derived from vegetable oils are one of the most suitable sources that have been used both historically and currently as raw materials for the production of materials from renewable resources [1-5]. Since almost all natural oils lack hydroxyl groups in their fatty acid structure, they must be chemically modified before they can be used, for example in polyurethane production. The fatty acids of vegetable oils can be converted into polyols using different techniques, such as epoxidation and oxirane ring opening, transesterification, amidisation, hydroformylation, ozonolysis, hydrothiolysis, etc [6]. The polyols obtained after the modifications have been used as component A in several studies to create polyurethane materials [7-12]. Traditionally, in addition to the above-mentioned component A (polyol), polyurethane materials are produced using component B, polyisocyanates, which are recognised as environmentally hazardous and highly toxic chemicals [13-17].

To develop polymers with similar properties to polyurethanes, the polymer industry needs alternative methods. The Michael addition process, which does not use isocyanates, makes it possible to produce polymer materials with properties comparable to those of polyurethane materials. The addition reactions of oxa-Michael [18, 19], thio-Michael [20-26], aza-Michael [23, 27-32] and carbon-Michael [33-35] can be used to create a polymer with a cross-linked polymer matrix structure [36]. More specifically, polyols can be converted to β-ketoesters via acetoacetylation to obtain the groups required for the Michael donor. Although the field of biopolymer development using α,β-unsaturated carbonyl compounds is not yet well explored, some studies have already shown that successful acetoacetylation of castor oil and castor oil-derived polyols [37-42] and soybean oil-derived polyols [43] can be achieved. Transacetoacetylation is most commonly performed with *tert-butyl* acetoacetate because it allows the reaction to be carried out in relatively mild conditions (110-130 °C) and is attractive from an industrial point of view due to its storage stability and wide commercial availability [37, 38, 40, 43-45].

From the resulting Michael donors (β-ketoesters), two-component thermosetting polymeric materials can be obtained by reaction with Michael acceptors (mainly alkylacrylates) in the presence of strong amine, amidine and guanidine-based catalysts [34-36, 40, 43, 46-49].

### Purpose and nature of the invention

The invention aims at the use of rapeseed oil for the production of Michael donors by conversion to acetoacetate esters via catalytic epoxidation/oxirane ring opening and acetoacetylation/transesterification. The acetoacetates obtained by this method are polyfunctional and can be used in Michael nucleophilic addition/polymerisation reactions to produce biopolymers. Depending on the chemical structure and functionality of the synthesised Michael donors, different polymeric materials with different cross-linking densities are obtained.

### Examples of how the invention has been achieved

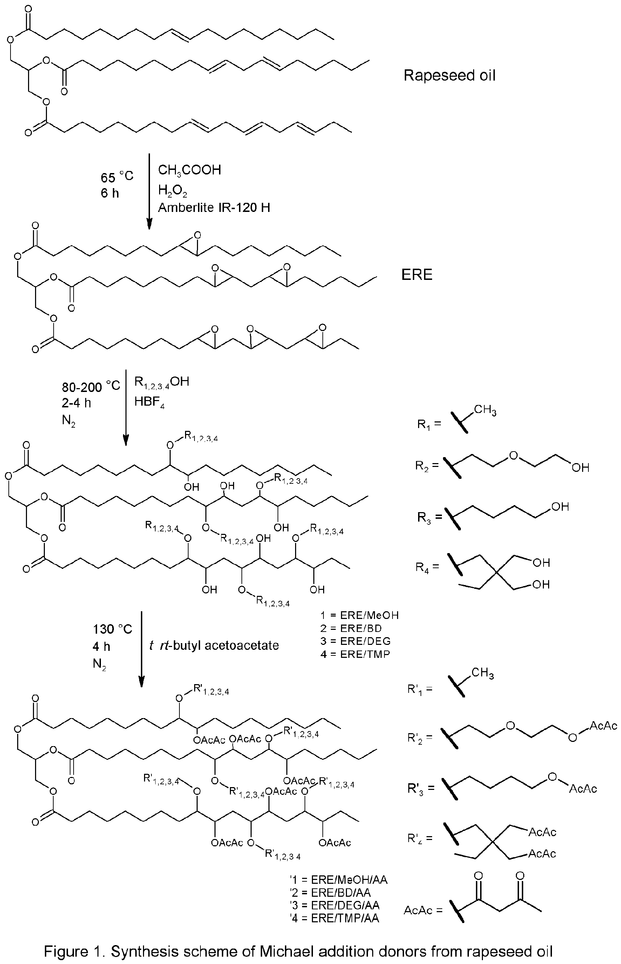

The epoxidation of rapeseed oil is achieved by *in situ* generated peroxyacetic acid, which is formed from acetic acid and hydrogen peroxide in the presence of an acidic catalyst. For this purpose, 800 g of rapeseed oil and 104 g of acetic acid are placed in a round four-necked flask into which ion exchange resin (e.g. Amberlite IR-120H, 20% of the amount of rapeseed oil) is added. The flask shall be equipped with a mechanical stirrer, a thermometer and a reflux condenser and placed in a thermostatic bath. As the flask warms up to 40 °C, a solution of 35% hydrogen peroxide in water (496 g) is added dropwise over a period of 30 minutes; the temperature of the contents of the flask must not exceed 65 °C during the reaction. After the addition of the entire quantity of hydrogen peroxide, the temperature of the entire contents of the flask must be maintained at 65 °C for at least six hours with continuous stirring at 600 rpm. The contents of the flask are then transferred to a separatory funnel and washed with water at 60 °C; after the water/organic part has stratified, they are separated. The organic part of the mixture is dried in a rotary vacuum evaporator until the residual water is completely removed. The synthesis produces an intermediate, epoxidised rapeseed oil (ERE) with an oxirane value of 3.80±0.10 mmol/g. The full synthesis scheme is shown in Figure 1.

The ERE obtained for the production of polyols is subjected to oxyrane ring opening with alcohols. The invention uses four alcohols with different functionalities - methanol (MeOH), 1.4-butanediol (BD), diethylene glycol (DEG) and trimethylolpropane (TMP). The opening of the oxirane rings and subsequent esterification with polyfunctional alcohols is carried out in a round four-neck flask equipped with a mechanical stirrer, reflux condenser, thermocouple and funnel. The flask is immersed in a bath of silicone oil and 100 g of ERE is added. A mixture of the appropriate alcohol and the catalyst tetrafluoroboric acid (0.25% by mass of ERE) is added dropwise to the ERE mixture. The synthesis mixture is stirred at 500 rpm. The temperature is kept constant, depending on the alcohol used. The amount of alcohol to be added and the synthesis temperature are shown in Table 1.

**Table 1.**

| Quantity of alcohol required for synthesis and synthesis temperature | | |
|---|---|---|
| Alcohol | Approximate quantity, g | Synthesis temperature, °C |
| MeOH | 11.5 | 80±5 |
| BD | 33.5 | 130±5 |
| DEG | 40.1 | 180±5 |
| TMP | 48.4 | 200±5 |

The synthesis results in four polyols of different functionality - ERE/MeOH, ERE/BD, ERE/DEG and ERE/TMP (Figure 1).

The synthesis of Michael donors from previously obtained polyols is carried out by acetoacetylation/transesterification with *tert-butyl acetoacetate.* For this purpose, *tert-butyl acetoacetate* is added dropwise to 150 g of the corresponding ERE polyol in a three-necked round-bottom flask in a molar ratio of 1:1 between the hydrosigroup and acetoacetate. The flask containing the mixture is immersed in a thermostatic water bath and the synthesis temperature is maintained at 130 °C. The evaporated *tert-butyl acetoacetate* is returned to the synthesis vessel by means of a Liebig condenser. Minimum synthesis time 2 hours, optimum synthesis time for maximum conversion 4 hours. This results in four different Michael donors (ERE/MeOH/AA - Sample 1, ERE/BD/AA - Sample 2, ERE/DEG/AA - Sample 3, ERE/TMP/AA - Sample 4), the structures of which are shown in Figure 1.

Prototypes against which the Samples are compared are acetoacetylated Michael donors derived from BASF Lupranol 3300 (1) and NEO Group Neopolyol 380 (2) polyols [50]. A comparison of the samples and prototypes in terms of their acid value, OH value and viscosity is shown in Table 2.

**Table 2.**

| Comparison of Prototypes and Samples | | | |
|---|---|---|---|
| | Acid value, mg KOH/g | Hydroxyl value, mg KOH/g | Viscosity, mPa·s |
| Prototype 1 | <5 | 26.2±0.4 | 211.1 |
| Prototype 2 | <5 | 40.7±0.6 | 814.0 |
| Sample 1 | <5 | 25.0±5.0 | 126.0 |
| Sample 2 | <5 | 50.0±5.0 | 105.3 |
| Sample 3 | <5 | 50.0±5.0 | 75.6 |
| Sample 4 | <5 | 60.0±5.0 | 503.7 |

The samples described in the invention differ from Prototype 1 and Prototype 2 by having higher hydroxyl values for Patterns 2, 3 and 4 and lower values for Pattern 1.

The samples described in the invention differ from Prototype 1 by having lower viscosity values for Patterns 1, 2 and 3, and a higher value for Pattern 4.

The samples described in the invention differ from Prototype 2 by having lower viscosity values for all samples.

Higher hydroxyl number samples are suitable feedstocks for polymeric materials as they allow the formation of products with a higher degree of cross-linking.

Lower viscosity Samples are of increased value for industrial applications as they facilitate their use in mechanical processes such as pumping, mixing and moulding.

### Information sources used

[1] Chen, R., Zhang, C., Kessler, M.R., 2015. Polyols and polyurethanes prepared from epoxidized soybean oil ring-opened by polyhydroxy fatty acids with varying oh numbers. J. Appl. Polym. Sci. 132, 1-10. doi:10.1002/app.41213
[2] Cifarelli, A., Boggioni, L., Vignali, A., Tritto, I., Bertini, F., Losio, S., 2021. Flexible polyurethane foams from epoxidized vegetable oils and a bio-based diisocyanate. Polymers (Basel). 13, 1-21. doi:10.3390/polym13040612
[3] Liao, Y.H., Su, Y.L., Chen, Y.C., 2021. The influence of neem oil and its glyceride on the structure and characterization of castor oil-based polyurethane foam. Polymers (Basel). 13. doi: 10.3390/polym13122020
[4] Sittinun, A., Pisitsak, P., Manuspiya, H., Thiangtham, S., Chang, Y.H., Ummartyotin, S., 2020. Utilization of Palm Olein-Based Polyol for Polyurethane Foam Sponge Synthesis: Potential as a Sorbent Material. J. Polym. Environ. 28, 3181-3191. doi:10.1007/s10924-020-01834-4
[5] Biermann, U., Bornscheuer, U., Meier, M.A.R., Metzger, J.O., Schäfer, H.J., 2011. Oils and fats as renewable raw materials in chemistry. Angew. Chemie - Int. Ed. 50, 3854-3871. doi:10.1002/anie.201002767
[6] Demirbas, A., 2011. Methylation of wood fatty and resin acids for production of biodiesel. Fuel 90, 2273-2279. doi:10.1016/j.fuel.2011.02.037
[7] Abolins, A., Pomilovskis, R., Vanags, E., Mierina, I., Michalowski, S., Fridrihsone, A., Kirpluks, M., 2021. Impact of different epoxidation approaches of tall oil fatty acids on rigid polyurethane foam thermal insulation. Materials (Basel). 14, 1-17. doi:10.3390/ma14040894
[8] Cao, Z., Gao, F., Zhao, J., Wei, X., Cheng, Q., Zhong, J., Lin, C., Shu, J., Fu, C., Shen, L., 2019. Bio-based coating materials derived from acetoacetylated soybean oil and aromatic dicarboxaldehydes. Polymers (Basel). 11. doi:10.3390/polym11111809
[9] Kirpluks, M., Vanags, E., Abolins, A., Michalowski, S., Fridrihsone, A., Cabulis, U., 2020. High functionality bio-polyols from tall oil and rigid polyurethane foams formulated solely using bio-polyols. Materials (Basel). 13, 38-53. doi:10.3390/MA13081985
[10] Sardari, A., Sabbagh Alvani, A.A., Ghaffarian, S.R., 2019. Castor oil-derived water-based polyurethane coatings: Structure manipulation for property enhancement. Prog. Org. Coatings 133, 198-205. doi:10.1016/j.porgcoat.2019.04.030
[11] Zhang, C., Madbouly, S.A., Kessler, M.R., 2015. Biobased polyurethanes prepared from different vegetable oils. ACS Appl. Mater. Interfaces 7, 1226-1233. doi:10.1021/am5071333
[12] Zhang, F.A., Lee, D.K., Pinnavaia, T.J., 2009. PMMA-mesocellular foam silica nanocomposites prepared through batch emulsion polymerization and compression molding. Polymer (Guildf). 50, 4768-4774. doi:10.1016/j.polymer.2009.08.007
[13] Dechent, S.E., Kleij, A.W., Luinstra, G.A., 2020. Fully bio-derived CO2 polymers for non-isocyanate based polyurethane synthesis. Green Chem. 22, 969-978. doi:10.1039/c9gc03488a
[14] Kathalewar, M., Sabnis, A., D'Mello, D., 2014. Isocyanate free polyurethanes from new CNSL based bis-cyclic carbonate and its application in coatings. Eur. Polym. J. 57, 99-108. doi:10.1016/j.eurpolymj.2014.05.008
[15] Kathalewar, M.S., Joshi, P.B., Sabnis, A.S., Malshe, V.C., 2013. Non-isocyanate polyurethanes: From chemistry to applications. RSC Adv. 3, 4110-4129. doi: 10.1039/c2ra21938g
[16] Lindsay, C.D., Timperley, C.M., 2020. TRPA1 and issues relating to animal model selection for extrapolating toxicity data to humans. Hum. Exp. Toxicol. 39, 14-36. doi: 10.1177/0960327119877460
[17] Sternberg, J., Pilla, S., 2020. Materials for the biorefinery: High bio-content, shape memory Kraft lignin-derived non-isocyanate polyurethane foams using a non-toxic protocol. Green Chem. 22, 6922-6935. doi:10.1039/d0gc01659d
[18] Jiang, Q., Zhang, Y.L., Du, Y., Tang, M., Jiang, L., Huang, W., Yang, H., Xue, X., Jiang, B., 2020. Preparation of hyperbranched polymers by oxa-Michael addition polymerization. Polym. Chem. 11, 1298-1306. doi:10.1039/c9py01686d
[19] Nising, C.F., Bräse, S., 2008. The oxa-Michael reaction: From recent developments to applications in natural product synthesis. Chem. Soc. Rev. 37, 1218-1228. doi:10.1039/b718357g
[20] Cespedes, S., Hand, R.A., Chmel, N., Moad, G., Keddie, D.J., Schiller, T.L., 2021. Enhanced properties of well-defined polymer networks prepared by a sequential thiol-Michael - radical thiol-ene (STMRT) strategy. Eur. Polym. J. 151, 110440. doi: 10.1016/j .eurpolymj.2021.110440
[21] Chatani, S., Nair, D.P., Bowman, C.N., 2013. Relative reactivity and selectivity of vinyl sulfones and acrylates towards the thiol-Michael addition reaction and polymerization. Polym. Chem. 4, 1048-1055. doi:10.1039/c2py20826a
[22] Chen, J., Ma, X., Edgar, K.J., 2021. A versatile method for preparing polysaccharide conjugates via thiol-michael addition. Polymers (Basel). 13, 1-13. doi: 10. 3 3 90/polym 13 121905
[23] Gunay, U.S., Cetin, M., Daglar, O., Hizal, G., Tunca, U., Durmaz, H., 2018. Ultrafast and efficient aza- and thiol-Michael reactions on a polyester scaffold with internal electron deficient triple bonds. Polym. Chem. 9, 3037-3054. doi:10.1039/c8py00485d
[24] Lenardão, E.J., Trecha, D.O., Ferreira, P. da C., Jacob, R.G., Perin, G., 2009. Green Michael Addition of Thiols to Electron Deficient Alkenes using KF/Alumina and Recyclable Solvent or Solvent-free Conditions. J. Braz. Chem. Soc. 20, 93-99. doi:10.1590/S0103-50532009000100016
[25] Li, Q.F., Chu, S., Li, E., Li, M., Wang, J.T., Wang, Z., 2020. Lanthanide-based hydrogels with adjustable luminescent properties synthesized by thiol-Michael addition. Dye. Pigment. 174, 108091. doi:10.1016/j.dyepig.2019.108091
[26] Piñeiro-García, A., Vega-Diaz, S.M., Tristán, F., Meneses-Rodríguez, D., Labrada-Delgado, G.J., Semetey, V., 2021. New insights in the chemical functionalization of graphene oxide by thiol-ene Michael addition reaction. FlatChem 26, 1-10. doi:10.1016/j.flatc.2021.100230
[27] Alaneed, R., Golitsyn, Y., Hauenschild, T., Pietzsch, M., Reichert, D., Kressler, J., 2021. Network formation by aza-Michael addition of primary amines to vinyl end groups of enzymatically synthesized poly(glycerol adipate). Polym. Int. 70, 135-144. doi: 10.1 002/pi.61 02
[28] Bhattacharjee, S., Shaikh, A.A., Ahn, W.S., 2021. Heterogeneous Aza-Michael Addition Reaction by the Copper-Based Metal-Organic Framework (CuBTC). Catal. Letters 151, 2011-2018. doi:10.1007/s10562-020-03459-7
[29] González, G., Fernández-Francos, X., Serra, A., Sangermano, M., Ramis, X., 2015. Environmentally-friendly processing of thermosets by two-stage sequential aza-Michael addition and free-radical polymerization of amine-acrylate mixtures. Polym. Chem. 6, 6987-6997. doi:10.1039/c5py00906e
[30] Mushtaque, M., Avecilla, F., Ahmad, I., Alharbi, A.M., Khan, P., Ahamad, S., Hassan, M.I., 2021. 5-Fluorouracil (5-FU)-based Aza-Michael addition product: A selective carbonic anhydrase IX inhibitor. J. Mol. Struct. 1231, 129977. doi:10.1016/j.molstruc.2021.129977
[31] Su, G., Thomson, C.J., Yamazaki, K., Rozsar, D., Christensen, K.E., Hamlin, T.A., Dixon, D.J., 2021. A bifunctional iminophosphorane squaramide catalyzed enantioselective synthesis of hydroquinazolines: Via intramolecular aza-Michael reaction to α,β-unsaturated esters. Chem. Sci. 12, 6064-6072. doi:10.1039/dlsc00856k
[32] Yang, X., Liu, H., Qian, L., Meng, Q., Wu, H., Li, Z., Zhou, H., 2021. Surface functionalization of cellulose fibers via aza-Michael addition for CO2-assisted water remediation. Appl. Surf. Sci. 554, 149593. doi:10.1016/j.apsusc.2021.149593
[33] Noordover, B., Liu, W., McCracken, E., DeGooyer, B., Brinkhuis, R., Lunzer, F., 2020. Michael addition curable coatings from renewable resources with enhanced adhesion performance. J. Coatings Technol. Res. 17, 1123-1130. doi:10.1007/s11998-020-00351-2
[34] Sonnenschein, M.F., Werness, J.B., Patankar, K.A., Jin, X., Larive, M.Z., 2016. From rigid and flexible foams to elastomers via Michael addition chemistry. Polymer (Guildf). 106, 128-139. doi:10.1016/j.polymer.2016.10.054
[35] Williams, S.R., Mather, B.D., Miller, K.M., Long, T.E., 2007a. Novel michael addition networks containing urethane hydrogen bonding. J. Polym. Sci. Part A Polym. Chem. 45, 4118-4128. doi:10.1002/pola.22236
[36] Mather, B.D., Viswanathan, K., Miller, K.M., Long, T.E., 2006. Michael addition reactions in macromolecular design for emerging technologies. Prog. Polym. Sci. 31, 487-531. doi:10.1016/j.progpolymsci.2006.03.001
[37] He, X., Zhong, J., Cao, Z., Wang, J., Gao, F., Xu, D., Shen, L., 2019. An exploration of the Knoevenagel condensation to create ambient curable coating materials based on acetoacetylated castor oil. Prog. Org. Coatings 129, 21-25. doi:10.1016/j.porgcoat.2018.12.015
[38] Trevino, A.S., Trumbo, D.L., 2002. Acetoacetylated castor oil in coatings applications. Prog. Org. Coatings 44, 49-54. doi:10.1016/S0300-9440(01)00223-5
[39] Wang, T., Wang, J., He, X., Cao, Z., Xu, D., Gao, F., Zhong, J., Shen, L., 2019. An ambient curable coating material based on the michael addition reaction of acetoacetylated castor oil and multifunctional acrylate. Coatings 9. doi: 1 0.3390/coatings90 1 0037
[40] Xu, D., Cao, Z., Wang, T., Zhong, J., Zhao, J., Gao, F., Luo, X., Fang, Z., Cao, J., Xu, S., Shen, L., 2019. An ambient-cured coating film obtained via a Knoevenagel and Michael addition reactions based on modified acetoacetylated castor oil prepared by a thiol-ene coupling reaction. Prog. Org. Coatings 135, 510-516. doi:10.1016/j.porgcoat.2019.06.026
[41] Zhu, Y., Gao, F., Zhong, J., Shen, L., Lin, Y., 2020. Renewable castor oil and DL-limonene derived fully bio-based vinylogous urethane vitrimers. Eur. Polym. J. 135, 109865. doi:10.1016/j.eurpolymj.2020.109865
[42] Zuo, H., Cao, Z., Shu, J., Xu, D., Zhong, J., Zhao, J., Wang, T., Chen, Y., Gao, F., Shen, L., 2019. Effect of structure on the properties of ambient-cured coating films prepared via a Michael addition reaction based on an acetoacetate-modified castor oil prepared by thiol-ene coupling. Prog. Org. Coatings 135, 27-33. doi:10.1016/j.porgcoat.2019.05.032
[43] Cao, Z., Gao, F., Zhao, J., Wei, X., Cheng, Q., Zhong, J., Lin, C., Shu, J., Fu, C., Shen, L., 2019. Bio-based coating materials derived from acetoacetylated soybean oil and aromatic dicarboxaldehydes. Polymers (Basel). 11. doi:10.3390/polym11111809
[44] Krall, E.M., Serum, E.M., Sibi, M.P., Webster, D.C., 2018. Catalyst-free ligin valorization by acetoacetylation. Structural elucidation by comparison with model compounds. Green Chem. 20, 2959-2966. doi:10.1039/c8gc01071d
[45] Witzeman, J.S., Nottingham, W.D., 1991. Transacetoacetylation with tert-Butyl Acetoacetate: Synthetic Applications. J. Org. Chem. 56, 1713-1718. doi:10.1021/jo00005a013
[46] Williams, S.R., Miller, K.M., Long, T.E., 2007b. Michael addition reaction kinetics of acetoacetates and acrylates for the formation of polymeric networks. Prog. React. Kinet. Mech. 32, 165-194. doi:10.3184/146867807X247730
[47] Aripo (bw, Es Sd, ;, St, S.L., Sz, T.Z., Euroasiática, Z.), Am, (, By, A.Z., Kg, K.Z., Tm ), 2014. W P Ct.
[48] Ozturk, G., Long, T.E., 2009. Michael addition for crosslinking of poly(caprolactone)s. J. Polym. Sci. Part A Polym. Chem. 47, 5437-5447. doi:10.1002/pola.23593
[49] Zhaoo, M.Y., Hsu, C.-P., Voeks, S.L., Landtiser, R., 2013. Acetoacetyl thermosetting resin for zero VOC gel coat.
[50] Pomilovskis, R.; Mierina, I.; Beneš, H.; Trhlíková, O.; Abolins, A.; Fridrihsone, A.; Kirpluks, M. The Synthesis of Bio-Based Michael Donors from Tall Oil Fatty Acids for Polymer Development. Polymers 2022, 14, 4107. https://doi.org/10.3390/polym14194107

## Claims

1. Production of Michael addition donors via acetoacetylation/transesterification of rapeseed oil derived polyols with *tert-butyl acetoacetate.*

2. The Michael addition donor production process according to Claim 1, wherein said rapeseed oil polyols are obtained from epoxidised rapeseed oil by oxyrane ring opening with polyfunctional alcohols.

3. The Michael addition donor derivative according to Claim 2, wherein said polyfunctional alcohols are methanol, 1.4-butanediol, diethylene glycol and trimethylolpropane.

4. The Michael addition donor production according to Claim 2, wherein said epoxidised rapeseed oil is obtained from rapeseed oil by its epoxidation with *in situ* generated peroxyacetic acid.
